# EUROPEAN PATENT APPLICATION

(11) **EP 2 529 711 A1**
(43) Date of publication of application: **05.12.2012**
(21) Application number: 12170477.9
(22) Date of filing: 01.06.2012
(51) Int. Cl.: A61F 5/56

(54) **A device for mandibular advancement**

(30) Priority: 02.06.2011 EP 11168610
(71) Applicant: Carash Industries Ltd, Worthing Sussex BN14 7QI (GB)
(72) Inventor: Ash, Simon, Worthing, West Sussex BN14 7QI (GB)
(74) Representative: Haley, Stephen

(57) **Abstract**

A device for advancing the lower jaw to an upper jaw in a mouth. The device comprises an upper member arranged to be retained on the upper jaw of a user in use.

A lower member is arranged to be retained on a lower jaw of a user in use and connection means is configured to connect the upper member to the lower member such that, in use, the lower jaw is advanced to and retained at a minimum distance relative to the upper jaw. The connection means is provided by at least one connecting member, each end of the connecting member being pivotably connected to one of the upper member and lower member. At least one of the pivotably connections is formed by a stud retained on the upper or lower member, the stud having a flange at the end which remains unconnected to the upper or lower member, the flange pivotably retaining, in use, an end of the connecting member on the upper or lower member.

## Description

The present invention relates to a device for advancing a lower jaw relative to an upper jaw by a predetermined distance and maintaining that distance. Such devices can be used for treating snoring and steep apnea without recourse to surgery. An example of such a device is shown in EP-A-2269543. In such a device, upper and lower members are arranged to be positioned and retained on the upper and lower jaws of a user and connection is provided between the two members such that the lower jaw is advanced and retained at a predetermined distance relative to the upper jaw when the device is being worn in use.

Whilst such a device has significant benefits in terms of improving the condition of the patient, the manufacture of such a device is not straightforward.

In particular, prior art devices such as the type listed above employ grub screws to attach components to one another, such screws being difficult to handle and having the potential to work lose during use, which can result in failure of the device and small components such as the screws to be ingested by a user. In addition, the connection points on such devices are in positions where the material forming the device is relatively thin, yet such connection points need to withstand further significant degrees of mechanical force whilst in use and therefore have the potential to break. In the past this has been solved by increasing the thickness of the device at the correction points, but this makes the points bulky and a potential irritant.

In addition, such devices often require a certain amount of assembly by a user, which can be difficult for the user if they have limited manual dexterity.

The present invention seeks to overcome the above and other problems.

According to the present invention there is provided a device for advancing the lower jaw to an upper jaw in a mouth, the device comprising:
an upper member arranged to be retained on the upper jaw of a user in use;
a lower member arranged to be retained on a lower jaw of a user in use; and connection means configured to connect the upper member to the lower member such that, in use, the lower jaw is advanced to and retained at a minimum distance relative to the upper jaw, wherein the connection means is provided by at least one connecting member, each end of the connecting member being pivotably connected to one of the upper member and lower member, wherein at least one of the pivotable connections is formed by a stud retained on the upper or lower member, the stud having a flange at the end which remains unconnected to the upper or lower member, the flange pivotably retaining, in use, an end of the connecting member on the upper or lower member, wherein the stud is attached to an embedding shaft embedded in the upper or lower member.

The stud may have a shaft which has a flattened key surface to receive a chetal end formed at one end of the connecting means.

The connecting member is formed from an arm and sleeve members and may have a retaining member formed therein to retain the arm within the sleeve. The retaining member may be provided by a slot and key arrangement formed in the respective ends of the sleeve and arm.

The device may be formed from stainless steel, a gold alloy, chrome cobalt, other metal, or a combination thereof.

The connecting means may be formed from two arm and sleeve members positioned on opposite sides of the device, and all of the ends of such piston and rod members may be retained by device stud configurations of the type defined above.

With the arrangement of the present invention it is possible to provide connections between the members and the connection means which are strong yet which do not protrude significantly from the device to cause irritation to the user. Furthermore, it is possible to provide connections which have no parts which can break away and have the potential for ingestion by the user. In addition, the connecting configuration of the present invention enables markings to be placed on the ends of the studs that are provided to provide information about the device in a simple and clearly legible manner.

A further advantage, when retaining members are provided in the piston and rod member(s), is that the entire device is retained in an assembled configuration and cannot fall apart even when not in use.

An example of the present invention will now be described with reference to the accompanying drawings, in which:
Figure 1 is a diagram showing a prior art device when positioned in the mouth of a user;
Figure 2 is a diagram showing the present invention.
Figure 3A and 3B are side views of studs employed in a device in accordance with the present invention;
Figures 4A to 4C show an alternative example connecting stud and components for use in the present invention;
Figure 5 show components that can be provided in the present invention to retain a sleeve and arm together in one example of the present invention; and
Figures 6A to 6D show components of a piston that may be used with the present invention.

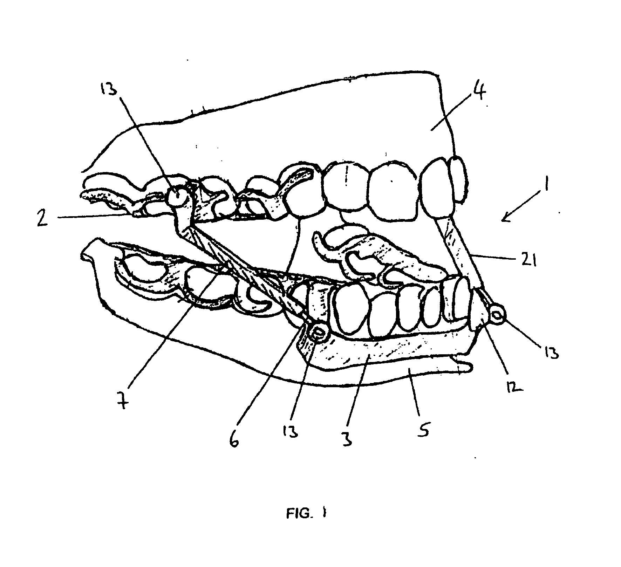

Figure 1 shows an example prior art device 1 comprising two main members, an upper member 2 and a lower member 3, which are configured to be fitted into the upper jaw 4 and lower jaw 4, respectively of a patients mouth. The upper and lower members 2, 3 of the device 1 are connected to such that the lower jaw 5 can move relative to the upper jaw 4 when the device 1 is fitted in a patient's mouth, but with the movement of the lower jaw 5 limited such that it is always advanced by a predetermined distance relative to the upper jaw 4.

In this device the upper and lower members 2, 3 are connected by a telescopic arm 6 and sleeve 7 forming a connecting member 21, such an arrangement is sometimes known as a "Herbst" telescope. Of course, it will be appreciated that alternative connecting arrangements could be provided.

The device 1 is usually formed of a cast metal, for example a gold alloy, but preferably chrome cobalt, which allows the device 1 to be compact but strong.

A mounting point 13 is provided on the upper member 2, and in this prior art device the mounting point is provided by a grub screw collet arrangement. A corresponding mounting point 13 is provided on the lower member 3. Again, in the prior art this has been provided by a grub screw collet arrangement. As can be seen more clearly in respect of the lower member 3, in the prior art in order to provide sufficient strength at the mounting point additional material 12 is provided in the relevant lower or upper member 3, 2.

As mentioned above, this prior art device, whilst working well in terms of the quality of treatment it provides, has a number of disadvantages. Firstly, the mounting points 13 can work loose and eventually the grub screws can fall out into the mouth of the user, rendering the device inoperable and leading to potential safety concerns as the user may be able to ingest the grub screws. Furthermore, the device is bulkier than perhaps it needs to be at the locations of the connecting points 13 in view of the additional supporting material 12 as is required. Another problem is that the components 6 and 7 can disengage with one another when the device 1 is removed from a user's mouth and reassembly of the components can be awkward.

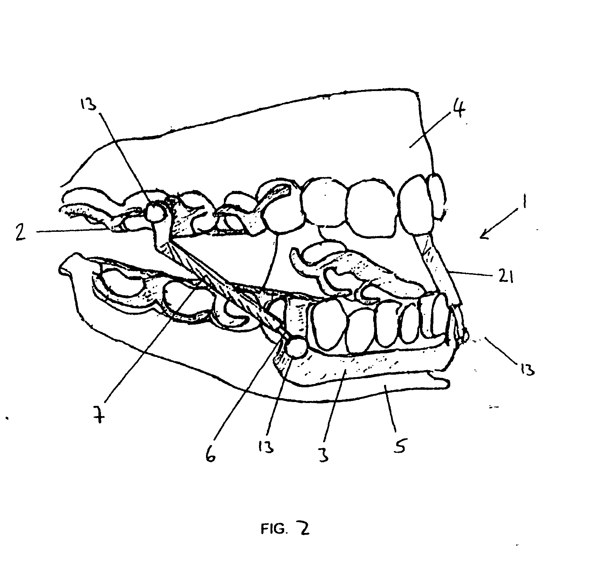

The present invention has the same overall general configuration as the prior art and is shown in figure 2, and components that which are identical to those in the prior art are numbered identically. In the present invention there is, again, an upper member 2 and lower member 3 which are connected by a connecting member and which can be positioned in the upper jaw 4 and lower jaw 5 respectively.

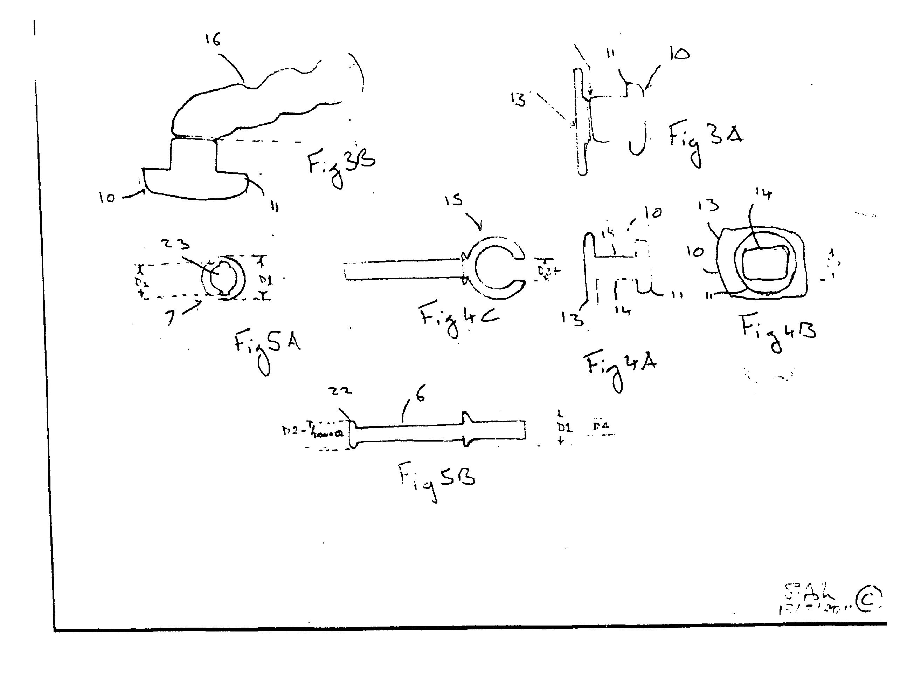

However, in the present invention the connecting points 13 are not provided by grub screw arrangements, rather they are provided by studs 10 of the type shown in figures 3A or 3B. The stud 10 has a retaining flange 11 at one end and is configured such that the other end of the stud can be attached to or embedded in one of the upper and lower members 2, 3. In the example shown in figure 3A, the stud is attached to a flange 13 which can be embedded in an upper or lower member 2, 3 to provide a strong yet low profile connection. In the example of figure 3A the stud may be provided by a round-headed stainless steel nail which is soldered or welded on after it has been passed through a retaining pivot formed on one of the arm 6 or sleeve 7 ends.

Figure 3B shows an alternative configuration in which an embedding shaft 16 is provided as an alternative to the provision of a flange for embedding in the upper or lower members 2, 3. This embedding shaft can be shaped so as to be positioned an optimum location in either the upper or lower member 2, 3 prior to that member being cast, so as to provide an exceptionally strong connection, yet maintaining minimum bulk at the connection point itself. Again, in this configuration a pivot connection is provided on the end of the connecting member and this is placed on the stud prior to it embedding in the upper or lower member 2, 3.

Figures 4A to 4C show an alternative arrangement. In this arrangement a stud 10 with retaining flange 11 is also provided. Again, an additional flange 13 is provided for embedding in either the upper or lower member 2, 3. However, in this case, as can be seen from the plan-cross sectional view of figure 4B, the main body 17 of the stud 10 has two flattened edges 14. These edges enable a chetal end 15 formed on either the arm or sleeve 6, 7 (see figure 4C) to pass around the main body of the stud 10 and then rotate around the stud to enable the appropriate pivoting. In function the chetal arm and stud arrangement forms a locking key between the piston rod arm and the stud connector inserted in the upper and lower frameworks. The benefit of this example over the earlier examples is that it is possible to place the connecting member on the device after casting of the upper and lower members 2, 3 by passing the chetal end 15 around the stud body 17.

Referring to figures 5A and B there is shown the components of the connecting member 21. Here the arm 6 of the connecting member 21 has a key 22 formed on one end, the key 22 being shaped so as to be inserted into a receiving opening 23 at one end of the sleeve 7 such that subsequent rotation of the arm 6 retains the arm 6 within the sleeve 7. In one example of the present invention such a structure is employed to provide the connecting member 21 between the upper 2 and lower 3 members of the device 1. Such a construction enables the rod 6 to move within the sleeve 7 so that a user can insert the device in their mouth and can move their lower jaw with respect to their upper jaw when the device 1 is in place. However, in addition, it also ensures that the rod 6 cannot fall out from within the sleeve 7 unintentionally. This means that, when the device 1 is removed from a user's jaw, the device 1 does not fall apart as it would with the prior art.

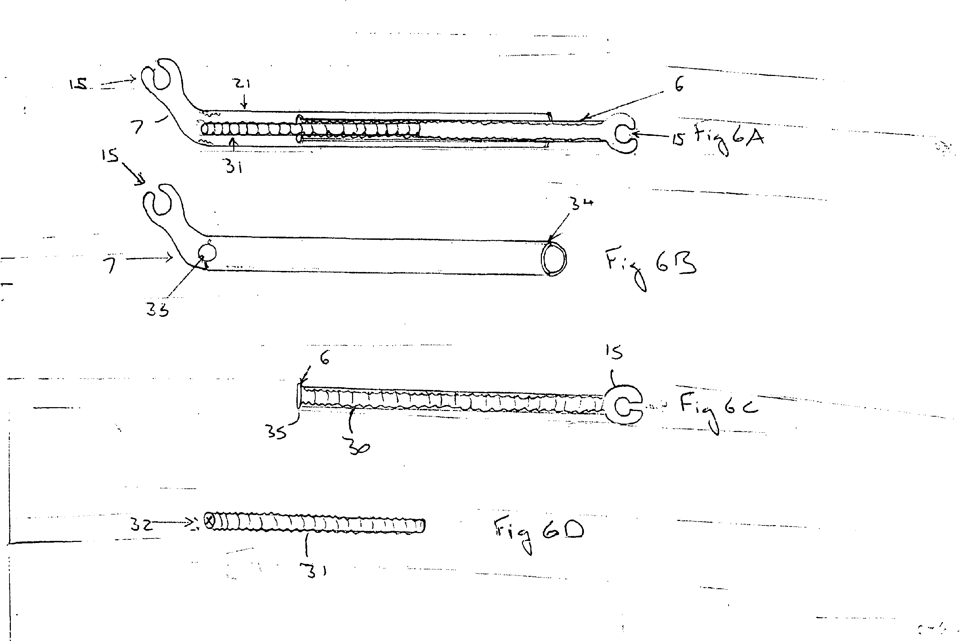

Figures 6A to 6D show an example connecting member 21 that can be used with the present invention. In this example, again, a chetal end 15 is again provided at each end of the connecting member 21 to connect with upper and lower members in use. Again, an arm 6 and sleeve 7 arrangement is provided, the arm 6 being retained within the sleeve 7 in use. However, in this example, the connecting member 21 has a range of extension which is adjustable so that it can be tailored to an individual user or adjusted over time to increase mandicular extension provided by the device or can be adjusted to reduce the length of the rod 6. The length of extension is provided by a combination of an inner thread 30 formed within the arm 6 and an engaging screw 31 that is positioned within the inner thread 30 in use. The head 32 of the screw thread 31 is configured so that it can engage with a screw driver and be turned to adjust its position within the arm 6 by movement along the inner thread 30. When the arm 6 is positioned within the sleeve 7 access to the head 32 of the screw 31 can be provided through a hole 33 in the sleeve 7. The hole 33 is smaller than the head 32 so that, when the connecting member 21 is fully compressed the head 32 engages with an inner surface of the sleeve 7 and prevents further insertion of the arm 6 into the sleeve 7. As with the example of figures 4 and 5, maximum extension of the arm 6 from within the sleeve 7 is controlled by engagement of retaining flanges 34, 35 on the sleeve 7 and arm 6 respectively. A configuration of the type shown in figure 5A may be used for the flange 34 on the sleeve 7 so that the arm 6 can be inserted into the sleeve 7 and then rotated such that the arm 6 is then retained within the sleeve 7 and cannot be removed without further rotation of the sleeve 7 with respect of the arm 6. The adjustable/adjustment parts are contained within the arm with controlled access via the hole 33.

As will be appreciated from the above description, the present invention provides a device which can be manufactured more reliably when compared to prior art devices whilst still providing the same level of treatment function to a user. In addition, it is more durable in use and has fewer safety issues associated with it. A further benefit is that the device can be used more easily without a user fearing that it will fall apart and it can be constructed so that it is more comfortable for a user during use.

## Claims

1. A device for advancing the lower jaw to an upper jaw in a mouth, the device comprising:
an upper member arranged to be retained on the upper jaw of a user in use;
a lower member arranged to be retained on a lower jaw of a user in use; and connection means configured to connect the upper member to the lower member such that, in use, the lower jaw is advanced to and retained at a minimum distance relative to the upper jaw, wherein the connection means is provided by at least one connecting member, each end of the connecting member being pivotably connected to one of the upper member and lower member, wherein at least one of the pivotable connections is formed by a stud retained on the upper or lower member, the stud having a flange at the end which remains unconnected to the upper or lower member, the flange pivotably retaining, in use, an end of the connecting member on the upper or lower member, and wherein the stud is attached to an embedding shaft which is embedded in the upper or lower member.

2. The device of claim 1, wherein the stud is a round headed nail which is soldered or welded on to the upper or lower member.

3. The device of any preceding claim in which the stud has a shaft which has a flattened key surface to receive a chetal end formed at one end of the connecting means.

4. The device of any preceding claim, where the connecting member is formed from an arm and sleeve and a retaining member formed therein to retain the arm within the sleeve.

5. The device of claim 4, wherein the retaining member is provided by a slot and key arrangement formed in respective ends of the sleeve and arm.

6. The device of claim 4 or claim 5 wherein a screw member is provided within the sleeve and is arranged to engage with the arm such that the screw member can be rotated to adjust the range of extension of the arm within the sleeve.

7. A device according to any preceding claim formed from stainless steel, a gold alloy, chrome cobalt, other metal or a combination thereof.

8. A device according to any preceding claim, wherein the connecting means is provided by two separate arm and sleeve configurations positioned on opposite sides of the device.
